# EUROPEAN PATENT SPECIFICATION

(11) **EP 0 623 351 B1**
(45) Date of publication and mention of the grant of the patent: **23.06.1999**
(21) Application number: 94106642.5
(22) Date of filing: 28.04.1994
(51) Int. Cl.: A61K 39/395, A61K 31/71

(54) **Topical pharmaceutical compositions containing antianthracycline monoclonal antibodies**
Topische pharmazeutische Zusammensetzungen, welche monoklonale Antikörper gegen Anthracycline enthalten
Compositions pharmaceutiques topiques contenant des anticorps monoclonaux antianthracyclines

(30) Priority: 04.05.1993 US 56382
(43) Date of publication of application: 09.11.1994
(73) Proprietor: ISTITUTO NAZIONALE PER LO STUDIO E LA CURA DEI TUMORI, I-20133 Milano (IT)
(72) Inventor: Ghione, Mario, I-20133 Milano (IT); Balsari, Andrea, I-20133 Milano (IT); Colnaghi, Maria Ines, I-20133 Milano (IT); Menard, Sylvie, I-20133 Milano (IT)
(74) Representative: Minoja, Fabrizio, Dr.

(56) References cited:
- EP-A- 0 439 048
- EP-A- 0 503 484

## Description

The present invention relates to topical pharmaceutical compositions comprising an antidotal effective amount of the anti-anthracycline antibiotic monoclonal antibody. These compositions are useful for decreasing the toxic effect caused by the administration of anthracycline antibiotics.

The invention particularly relates to the use of anti-anthracycline monoclonal antibodies or fragments thereof for the preparation of topical medicaments useful for preventing anthracycline-induced alopecia.

The term fragment, as used herein, means for instance Fab chain.

Anthracycline antibiotics are a widely investigated class of compounds having antitumor activity. Particularly, doxorubicin (DXR) is being used for a long time in antitumor chemotherapy protocols.

The clinical usefulness of anthracycline antibiotics is limited by serious side effects such as cardiomyophaty, bone-marrow depression, gastrointestinal tract mucositis and, if there is drug leakage at the injection site, local tissue necrosis.

Another peculiar side effect consists in alopecia, which is due to the inhibiting activity exerted by anthracyclines on the continuously growing cells of hair roots. Alopecia occurs in almost the totality of the patients treated with anthracycline drug.

Although not life-threatening, drug-induced alopecia can be psychologically devastating for a patient and may lead to refusal of chemotherapy. Clearly, its prevention has implications for improving patient adaption to chemotherapy. Attempts to eliminate this psychologically and socially traumatic side effect by using tourniquets or hypothermia to reduce scalp blood flow have met with little success, as both approaches are effective only with low-dose anthracycline treatment. EP-A-503484 teaches the use of anti-anthracycline antibodies for the reduction of anthracycline side-effects.

Now it has surprisingly been found that anti-anthracycline monoclonal antibodies (MAbs) and particularly anti-doxorubicin antibodies, when topically applied, effectively prevent hair loss induced by anthracycline administration.

It is particularly surprising that monoclonal antibodies are able of penetrating the stratum corneum of the skin without losing their function.

According to the present invention, anti-anthracycline MAbs, prepared as described in EP-A-0316776 and Int. J. Cancer 42, 798-802, 1988 and Anticancer Res. 10, 129-132, 1990, from the hybridoma deposited at ECACC under no. 90011003 on January 12, 1990, are administered to patients affected by tumors which are sensitive to treatment with anthracyclines, preferably before the anthracycline antibiotic treatment, by means of a suitable topical carrier.

According to a preferred embodiment, the invention provides a pharmaceutical preparation containing anti-anthracycline MAb entrapped in liposomes for topical administration for the prevention of alopecia. Briefly, the liposomes may be prepared according to well-known methods, for instance by mixing chloroform solutions of phosphatidylcholine, phosphatidylserine, cholesterol or analogous formulations. The organic solvent is then removed by evaporation, and a MAb solution at a concentration of, for example, from 0.1 to 1 mg/ml is added to the dried lipid film. The preparation of multilamellar vesicles is preferred and is made easier by vortex dispersion and further sonication.

The loading of liposomes with the MAb can be carried out according to methods already described for the preparation of so-called immunoliposomes (see "Medical application of liposomes", K. Yagy ed. Darger AG, Base 1986). The obtained liposomes are then advantageously formulated in administration forms, such as gels, ointments, lotions and the like, according to known methods.

The concentration of the monoclonal antibody in said administration form may range from 0.01 mg to 1 mg per ml of the final administration form. The treatment schedule will generally consist of the application of said administration form contemporaneously or from 120 to 30' before each anthracycline antibiotic administration, so as to cover the whole scalp surface.

The following examples further illustrate the invention.

### Example 1

### Preparation of liposome-entrapped anti-doxorubicin (DXR) monoclonal antibody

The anti-DXR monoclonal antibody secreted by the hybridoma deposited at ECACC under no. 90011003 hereinafter referred to with the abbreviation MADll, was purified by affinity chromatography on a protein A-Sepharose CL413 column.

The purified monoclonal antibody was incapsulated into multilamellar liposomes prepared by sonication of a phosphatidylcholine, phosphatidylethanolamine and phosphatidylinositol mixture (1:1:1 molar ratio).

A final concentration of 1 mg of antibody per milliliter of liposome was obtained.

### Example 2

### Prevention of doxorubicin-induced alopecia in rats

In the first experiment, 20 8-day old rats, randomly divided into two groups, were injected i.p. with 2 mg . kg body weight⁻¹. day⁻¹ for 7 days of DXR, and 2 h before every drug injection, treated or untreated on the head and proximal neck with 100 µl of MAD11-loaded liposomes of Example 1 (Table 1, Expt. I). All animals had an almost full coat of fur at day 6 of the experiment. On day 14,9 of 10 rats of the group treated only with DXR showed severe head and neck alopecia. In rats treated with DXR plus MADll, severe alopecia was observed in one rat, two showed mild alopecia, and six rats showed no alopecia. One rat of this group died on day 12.

In a second experiment, 30 8-day old rats were randomly divided; the abdomen of rats was topically treated with 100 µl MADll-loaded or unloaded liposomes 2 h before i.p. injection with a single dose of DXR (4 mg/kg) (Table 1, Expt. II). On day 9, 9 of 15 rats treated with DXR showed severe abdominal alopecia, 2 had mild alopecia, and the remaining 4 showed no alopecia. In rats treated with DXR plus MADll, severe alopecia was observed in only 1 rat, 3 rats had mild alopecia, and 11 rats showed no alopecia. Inhibition of alopecia was permanent in the MADll treated rats; after 30 days, alopecia had reversed in all rats. In a third experiment, two groups of rats were treated i.p. with 2.8 mg/kg of DXR every second day for a total of three injections. The abdomens of one group (21 rats) were treated topically with liposomes loaded with the anti-DXR MAb 2 h before each DXR treatment. The second group (18 rats) received the same treatment, but with liposomes loaded with a control MAb directed against the human HER2/neu oncoprotein. On day 10 of the experiment (5 days after the last DXR treatment), 11 rats in the second group had severe alopecia, 3 had mild alopecia, and 4 rats had no alopecia, whereas in the MADll treated group alopecia was completely absent in 14 animals, and only mild in 6; only 1 rat presented severe alopecia. Histological examination of skin biopsies showed a noticeable loss of hair follicles in unrelated MAb treated rats, whereas MADll treated rats without alopecia showed no damage of the follicles.

The results of the three experiments, are Summarized in the following table:

| Group | Number of mice with alopecia | | |
|---|---|---|---|
| | Negative | Mild | Severe |
| | | | |

| Expt. I | | | |
|---|---|---|---|
| DXR + MADll | 6 | 2 | 1 |
| DXR | 1 | - | 9 |

| Expt. II | | | |
|---|---|---|---|
| DXR + MADll | 11 | 3 | 1 |
| DXR | 4 | 2 | 9 |

| Expt. III | | | |
|---|---|---|---|
| DXR + MADll | 14 | 6 | 1 |
| DXR + unrelated MAb | 4 | 3 | 11 |

The difference between the group treated with DXR or with DXR plus unrelated MAb and that with DXR plus MADll was significant (X²-test analysis: P' 0.003, Expt. I; P' 0.008, Expt. II; and P' 0.001 Expt. III).

Immunohistochemistry and histopathology tests showed the penetration of the labelled antibody into the stratum corneum of the skin and its persistance at the site of application for several hours, with negligible systemic absorption.

Preliminary clinical tests have confirmed the results obtained in the animal tests.

## Claims

1. The use of anti-anthracycline monoclonal antibodies or fragments thereof for the preparation of a topical medicament useful for preventing anthracycline-induced alopecia.

2. The use according to claim 1 of the antibody secreted by the hybridoma deposited at ECACC under no. 90011003.

3. The use according to claim 1 or 2 wherein the monoclonal antibody is delivered by liposomes.

4. A topical pharmaceutical composition containing as the active principle an anti-anthracycline monoclonal antibody in admixture with a suitable carrier.

5. A topical pharmaceutical composition according to claim 4 in form of liposomes.

6. A topical pharmaceutical composition according to claim 5 in form of multilamellar liposomes.

7. A topical pharmaceutical composition according to any one of claims 4 to 6 containing the anti-doxorubicin antibody secreted by the hybridoma deposited at ECACC under no. 90011003.

## Patentansprüche

1. Die Verwendung von Anti-Anthracyclin-monoklonalen Antiköpern oder Fragmenten hiervon zur Herstellung eines Medikaments zur äußerlichen Anwendung zur Verhinderung von Anthracyclin-induzierter Alopecia.

2. Die Verwendung gemäß Anspruch 1 des Antiköpers, sekretiert durch das Hybridoma, hinterlegt bei ECACC unter der Nr. 90011003.

3. Die Verwendung gemäß Anspruch 1 oder 2, wobei der monoklonale Antikörper durch Liposomen freigesetzt wird.

4. Eine pharmazeutische Zusammensetzung zur äußerlichen Anwendung, enthaltend als einen aktiven Bestandteil einen Anti-Anthracyclin-monoklonalen Antikörper in einer Mischung mit einem geeigneten Träger.

5. Eine pharmazeutische Zusammensetzung zur äußerlichen Anwendung gemäß Anspruch 4 in Form von Liposomen.

6. Eine pharmazeutische Zusammensetzung zur äußerlichen Anwendung gemäß Anspruch 5 in Form von multilamellaren Liposomen.

7. Eine pharmazeutische Zusammensetzung zur äußerlichen Anwendung gemäß einem der Ansprüche 4 bis 6, enthaltend den Anti-Doxorubicin-Antikörper, sekretiert durch das Hybridoma, hinterlegt bei der ECACC unter der Nr. 90011003.

## Revendications

1. Utilisation d'anticorps monoclonaux anti-anthracycline ou leurs fragments pour la préparation d'un médicament topique utile dans la prévention de l'alopécie induite par une anthracycline.

2. Utilisation selon la revendication 1 de l'anticorps sécrété par l'hydridome déposé chez ECACC sous le n° 90011003.

3. Utilisation selon la revendication 1 ou 2, dans laquelle l'anticorps monoclonal est délivré par des liposomes.

4. Composition pharmaceutique topique contenant comme principe actif un anticorps monoclonal anti-anthracycline en mélange avec un véhicule approprié.

5. Composition pharmaceutique topique selon la revendication 4 sous forme de liposomes.

6. Composition pharmaceutique topique selon la revendication 5 sous forme de liposomes multilamellaires.

7. Composition pharmaceutique topique selon l'une quelconque des revendications 4 à 6, contenant l'anticorps anti-doxorubicine sécrété par l'hybridome déposé chez ECACC sous le n° 90011003.
